(19) Europäisches Patentamt
European Patent Office
Office européen des brevets

(11) EP 2 215 459 B1

(12) EUROPÄISCHE PATENTSCHRIFT

(45) Veröffentlichungstag und Bekanntmachung des Hinweises auf die Patenterteilung:
02.10.2013 Patentblatt 2013/40

(21) Anmeldenummer: 08857948.7

(22) Anmeldetag: 15.11.2008

(51) Int Cl.:
G01N 33/24 (2006.01)      G01N 22/04 (2006.01)
A01G 25/16 (2006.01)      G01N 33/00 (2006.01)

(86) Internationale Anmeldenummer:
PCT/DE2008/001896

(87) Internationale Veröffentlichungsnummer:
WO 2009/071047 (11.06.2009 Gazette 2009/24)

(54) **Verfahren und Vorrichtung zur Bestimmung von Biomasse mittels eines Mikrowellenresonators**

Method and device for determining biomass by means of a microwave resonator

Procédé et dispositif de détermination de biomasse à l'aide d'un résonateur micro-ondes

(84) Benannte Vertragsstaaten:
AT BE BG CH CY CZ DE DK EE ES FI FR GB GR HR HU IE IS IT LI LT LU LV MC MT NL NO PL PT RO SE SI SK TR

(30) Priorität: 05.12.2007 DE 102007058836

(43) Veröffentlichungstag der Anmeldung:
11.08.2010 Patentblatt 2010/32

(73) Patentinhaber: Forschungszentrum Jülich GmbH
52425 Jülich (DE)

(72) Erfinder:
• BLÜMLER, Peter
55263 Wackernheim (DE)
• MENZEL, Marion, Irene
81927 München (DE)
• KRAUSE, Hans-Joachim
52499 Baesweiler (DE)
• GILMER, Frank
67063 Ludwigsburg (DE)

(56) Entgegenhaltungen:
WO-A-00/14552        DE-A1- 2 552 954
DE-A1- 19 710 591

• KRASZEWSKI A W ET AL: "Microwave resonator for sensing moisture content and mass of single wheat kernels" CANADIAN AGRICULTURAL ENGINEERING, OTTAWA, CA, Bd. 36, Nr. 4, 1. Oktober 1994 (1994-10-01), Seiten 231-238, XP008104810 ISSN: 0045-432X

• HONG S CHUA ET AL: "An Instrument to Measure the Characteristics of Single Wheat Grain Kernels" MICROWAVE CONFERENCE, 2006. 36TH EUROPEAN, IEEE, PI, 1. September 2006 (2006-09-01), Seiten 1402-1405, XP031005848 ISBN: 978-2-9600551-6-0

• WEILIAN WANG ET AL: "Precise Diagnosis of Water Stress in Plants Based on Microwave Sensor"INFORMATION ACQUISITION, 2006 IEEE INTERNATIONAL CONFERENCE ON, IEEE, PI, 1. August 2006 (2006-08-01), Seiten 1163-1167, XP031045333 ISBN: 978-1-4244-0528-2

• CALLA O P N ET AL: "Comparative study of the methods of measurement of dielectric constant at microwave frequencies for dry and wet soil" INDIAN JOURNAL OF RADIO AND SPACE PHYSICS, NATIONAL INSTITUTE OF SCIENCE COMMUNICATION AND INFORMATION RESOURCES, Bd. 32, Nr. 2, 1. April 2003 (2003-04-01), Seiten 108-113, XP008105003 ISSN: 0367-8393

• JONES C. L., STONE M. L., MANESS N. O., SOLIE J. B., BRUSEWITZ G. H.: "Plant Biomass Estimation Using Dielectric Properties" PAPER NUMBER 063092, 2006 ASAE ANNUAL MEETING, 2006, XP008104979

• KRAUSE H-J ET AL: "Dielectric microwave resonator for non-destructive evaluation of moisture and salinity" 9TH EUROPEAN CONFERENCE ON NDT: ECNDT BERLIN 2006; SEPTEMBER 25 - 29, 2006, EUROPEAN FEDERATION FOR NON-DESTRUCTIVE TESTING, BERLIN, 29. September 2006 (2006-09-29), Seiten 1-12, XP008104806 ISBN: 978-3-931381-86-8

- **FERRAZZOLI P ET AL: "Sensitivity to microwave measurements to vegetation biomass and soil moisture content: a case study" IEEE TRANSACTIONS ON GEOSCIENCE AND REMOTE SENSING, IEEE SERVICE CENTER, PISCATAWAY, NJ, US, Bd. 30, Nr. 4, 1. Juli 1992 (1992-07-01), Seiten 750-756, XP007908100 ISSN: 0196-2892**

**Beschreibung**

Die Erfindung betrifft ein Verfahren zur Bestimmung von Biomasse mittels dielektrischer Messungen im Mikrowellenresonator sowie eine dazu geeignete Vorrichtung.

Traditionell erfolgt die Bestimmung der Biomasse über die gravimetrische Bestimmung der Trockenmasse pro Volumeneinheit.

Die bisher bekannten Verfahren weisen den Nachteil auf, dass sie zum einen nur mit einer vollständigen Entnahme bzw. Zerstörung der untersuchten Probe/Biomasse einhergehen (invasives Verfahren). Weiterhin ist es von Nachteil, dass die Bestimmung aufgrund der erforderlichen Trocknung relativ zeitaufwendig ist.

Die Veröffentlichung "Microwave resonator for sensing moisture content and mass of single wheat kernels" von Kraszewski W. et al. in der Zeitschrift Canadian Agricultural Engineering, Bd. 36, Nr. 4, 1. Oktober 1994, Seiten 231 - 238, offenbart ein Verfahren zur Bestimmung von Biomasse, wobei die polarisierte Biomasse in den Innenraum eines Mikrowellenresonators eingebracht wird, die veränderten Resonanzeigenschaften gemessen werden und die Biomasse an Hand von kalibrierten Daten ermittelt wird.

Es ist daher Aufgabe der Erfindung, ein schnelles, nicht-invasives Verfahren zur Bestimmung von Biomasse zu schaffen. Es ist weiterhin Aufgabe der Erfindung, eine Vorrichtung bereit zu stellen, mit der eine schnelle, nicht-invasive Bestimmung von Biomasse erreicht werden kann.

Ausgehend vom Oberbegriff des Anspruchs 1 wird die Aufgabe erfindungsgemäß gelöst mit den im kennzeichnenden Teil des Anspruchs 1 angegebenen Merkmalen. Weiterhin wird die Aufgabe ausgehend vom Oberbegriff des Anspruchs 6 erfindungsgemäß gelöst mit den im kennzeichnenden Teil des Anspruchs 6 angegebenen Merkmalen.

Es hat sich gezeigt, dass mit dem erfindungsgemäßen Verfahren eine nicht-invasive schnelle Überwachung (Screening) des Wachstums von Biomasse möglich wird. Weiterhin wird eine Verteilung der Biomasse im Höhenprofil erfasst. Hierzu wird die Biomasse zur Messung in den Resonatorraum eingeführt oder der Resonator entlang der Pflanzenachse geführt. In der erfindungsgemäßen Vorrichtung ist der Resonator für diese Anwendung entlang der Längsachse aufklappbar ausgestaltet, um ein einfaches Messen der Biomasse zu ermöglichen.

Mit dem erfindungsgemäßen Verfahren wird es beispielsweise auch möglich, über einen längeren Zeitraum mehrmals an identischen Pflanzen den Zuwachs der Biomasse direkt zu verfolgen.

Der Resonator kann einen kreisförmigen oder auch einen eckigen Querschnitt aufweisen. Das erfindungsgemäße Verfahren gemäß Anspruch 1 betrifft ein Verfahren zur Bestimmung von Biomasse mittels dielektrischer Messungen im Mikrowellenresonator, bei dem die polarisierbare Biomasse in den Innenraum eines Mikrowellenresonators eingebracht wird.

Unter der Bezeichnung Biomasse soll in Rahmen der vorliegenden Anmeldung organisches Material, wie z. B. Pflanzenmaterial, verstanden werden.

Zur Messung wird beispielsweise der oberirdische Teil der Pflanze in einen Resonator eingebracht oder von ihm umhüllt. Der Bereich des Wurzelraums mit dem Pflanzsubstrat sollte in diesem Fall vor dem Resonator abgeschirmt werden. Als Abschirmung ist jedes elektrisch leitfähige Material geeignet. Dieses kann als Vollmaterial oder auch beispielsweise als Netz ausgestaltet sein. In der netzförmigen Ausgestaltung der Abschirmung kann die Maschenweite beispielsweise ca. 1/20 der Wellenlänge (ca. 10 mm bei 1 GHz mit 200 mm Wellenlänge) betragen.

Das Messverfahren basiert auf der Änderung der dielektrischen Eigenschaften des Resonator-Innenraums durch Einbringen polarisierbaren Materials, wie z. B. Wassers (Polarisierung der Wassermoleküle in einem von außen angelegten elektromagnetischen Feld). Das Wasser bewirkt eine Frequenzverschiebung und eine Änderung der Güte (= Dämpfung bei der Peakbreite von Transmissionsminimum -3 dB) in einem Mikrowellen-Resonator. Auf Grund wasserinterner Bindungskräfte entsteht bei zunehmender Anregungsfrequenz im MHz-Bereich aufgrund von Trägheit in der Polarisation zwischen den Molekülen und dem äußeren Feld eine Phasenverschiebung, welche zunehmend dielektrische Verluste bewirkt. Insbesondere bei organischen Substanzen und biologischem Gewebe werden die dielektrischen Verluste wichtig. Mittels dieses dielektrischen Messverfahrens lassen sich bereits kleine Wassermengen im ml-Bereich sowohl in organischem, wie auch in anorganischem Material, detektieren. Da Pflanzen zum größten Teil aus Wasser bestehen (80-90% Gewichtsanteil) ist dieses Verfahren zur nicht-invasiven Biomassen (= Wasser-Bestimmung) an Pflanzen besonders geeignet.

Mit Hilfe einer Kalibrierung über die Verschiebung der Resonanzfrequenz und/oder Änderung der Güte der Resonanzeigenschaften in einer eingewogenen Biomasseprobe kann die absolute Menge an Wasser bestimmt werden. Ist diese dann bekannt, können Trockenmasse, relativer Wassergehalt und relativer Anteil Trockenmasse wie folgt berechnet werden:

| Trockenmasse (g) | = Einwaage (g) - Wasser(g) |
| Relativer Wassergehalt (%) | = Wasser (g) / Einwaage (g) * 100 |
| Relativer Anteil Trockenmasse (%) | = [Trockenmasse (g) / Einwaage (g)] * 100 |

Die Kalibrierung ist für jede Pflanzenart spezifisch. Mittels einer Datenbank können die kalibrierten Werte gespeichert und dann je nach Bedarf abgerufen werden, ohne dass eine neue Kalibrierung erforderlich wird.

Es kann in unterschiedlichen Moden des Resonators gemessen werden. Der Frequenzbereich der Moden ist abhängig vom jeweiligen Resonator. So liegt der Bereich der unteren Moden beispielsweise für den im Ausführungsbeispiel verwendeten Resonator im Bereich von 750 bis 900 MHz für die erste Mode und für die zweite Mode von 1150 bis 1350 MHz. Die Messdauer pro Probenmessung kann dabei im Sekundenbereich liegen.

Neben der Bestimmung des Wassergehalts ist es weiterhin auch möglich, andere Parameter, wie z. B. den Salzgehalt der Biomasse, zu untersuchen.

So eignen sich beispielsweise niedrige Moden von 1 bis 2 besonders gut zur Messung des Wassergehalts. Moden >2 können besonders geeignet sein zur Messung des Salzgehalts. Der Realanteil des Messsignals beschreibt die Verschiebung der Resonanzfrequenz und korreliert mit dem Wassergehalt. Der Imaginäranteil beschreibt die Phasenverschiebung der Resonanzfrequenz und korreliert mit dem Salzgehalt. Jede Mode zeichnet sich durch eine charakteristische räumliche Empfindlichkeitsverteilung aus, die sich aus dem spezifischen Feldlinienbild der Mode ableiten lässt. Durch die Kombination der gewonnenen Informationen aus der Messung mehrerer Moden lassen sich einerseits Variationen in den Messsignalen aufgrund unterschiedlicher Biomasseverteilungen reduzieren, andererseits prinzipiell auch Rückschlüsse auf diese Biomasseverteilung ziehen.

Eine Auswerteelektronik, z. B. ein Netzwerkanalysator, ermittelt die Resonanzeigenschaften, übermittelt diese an eine elektronische Datenverarbeitungseinheit, mit der dann ein Abgleich mit den bereits kalibrierten Daten einer Datenbank durchführt werden kann.

In einer vorteilhaften Ausführung der Erfindung wird die Resonatorgeometrie an die zu untersuchende Pflanzenwuchsform angepasst. Die Resonatordimensionierung erfolgt je nach Bedarf entsprechend dem zu untersuchenden Gut/Pflanzenmaterial. Seine Resonanzfrequenz ist, bei gegebenen Dielektrizitäts- und Permeabilitätskonstanten (abhängig vom Messobjekt) umgekehrt proportional zum Innendurchmesser.

Die Erfindung betrifft weiterhin eine Vorrichtung, die für das oben beschriebene Verfahren geeignet ist.

Vorteilhafte Weiterbildungen sind in den Unteransprüchen angegeben.

Im Folgenden sollen einige nicht zum Erfindungsgegenstand gehörende Ausführungsbeispiele angegeben werden, die das Messprinzip näher erläutern.

Die Figuren zeigen:

Fig. 1:  Geometrie eines Resonators
Fig. 1a:  Graphische Darstellung der Definition der Achsen im Resonator
Fig. 2:  Resonanzfrequenzverlauf der ersten Mode im Resonator

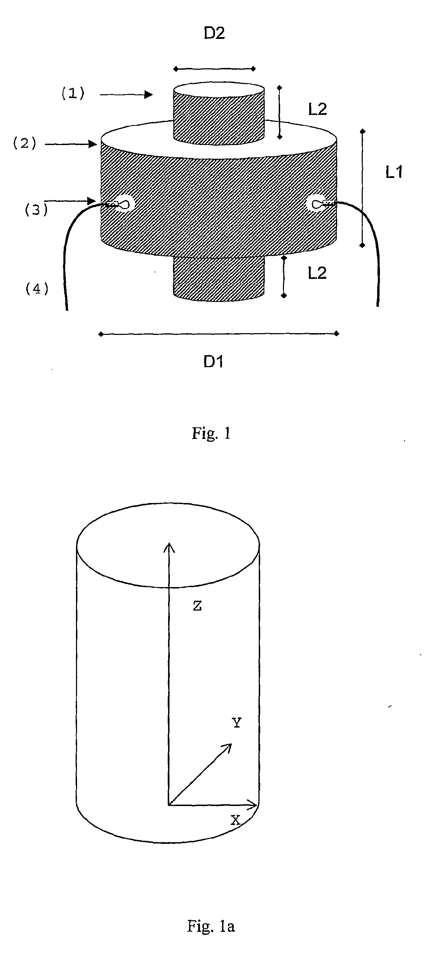

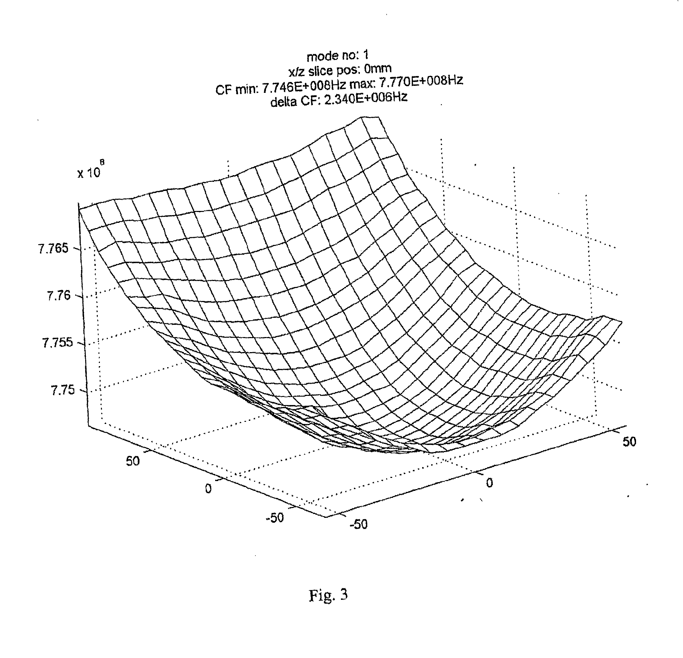

Bei den Figuren 3 bis 13 geben die horizontalen Achsen, welche die horizontale Ebene der Figur bilden, die jeweils untersuchten Positionen der X/Y-Achse bzw. X/Z-Achsen im Resonator (s. Fig 1a) in (mm) an, wobei die Position 0 (mm) dem Zentrum des Resonators entspricht. Die senkrecht zu der Ebene aufgezeigte Achse gibt die Werte der gemessenen Resonanzfrequenz [f] in (Hz) wieder.

Fig. 3: Vertikaler Verlauf (entlang der Z-Achse) der Resonanzfrequenz in der 1. Mode. Horizontale Ebene der Figur entspricht den untersuchten Positionen in der X/Z-Achse

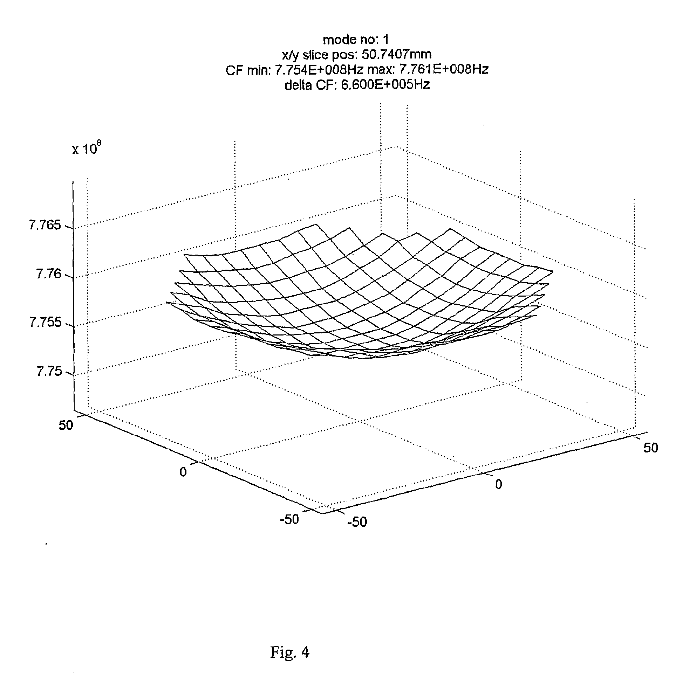

Fig. 4: Horizontaler Verlauf (entlang der X/Y-Ebene) der Resonanzfrequenz in der 1. Mode bei einer Höhe der Z-Achse im Resonator von 50 mm (0 mm = Zentrum des Resonators). Horizontale Ebene der Figur entspricht den untersuchten Positionen in der X/Y-Achse.

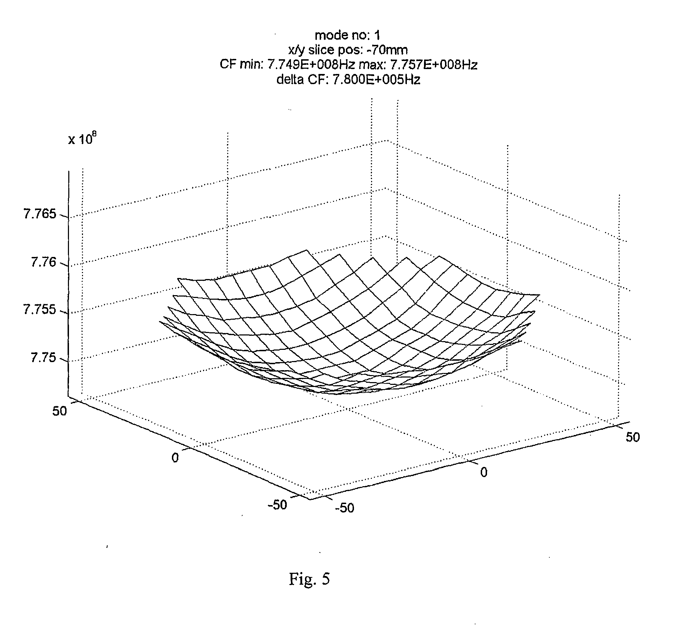

Fig. 5: Horizontaler Verlauf (entlang der der X/Y-Ebene des Resonators) der Resonanzfrequenz bei einer Höhe der Z-Achse im Resonator von -70 mm in der 1. Mode. Horizontale Ebene der Figur entspricht den untersuchten Positionen in der X/Y-Achse.

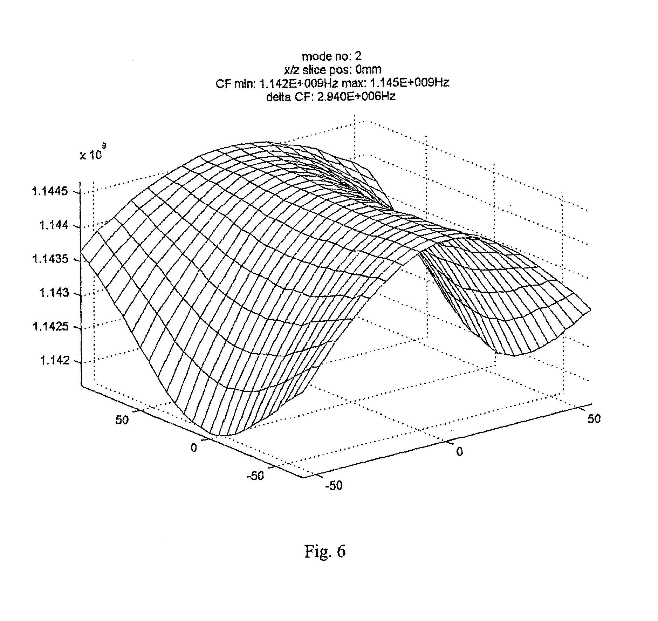

Fig. 6: Vertikaler Verlauf (entlang der Z-Achse) der Resonanzfrequenz in der 2. Mode. Horizontale Ebene der Figur entspricht den untersuchten Positionen in der X/Z-Achse.

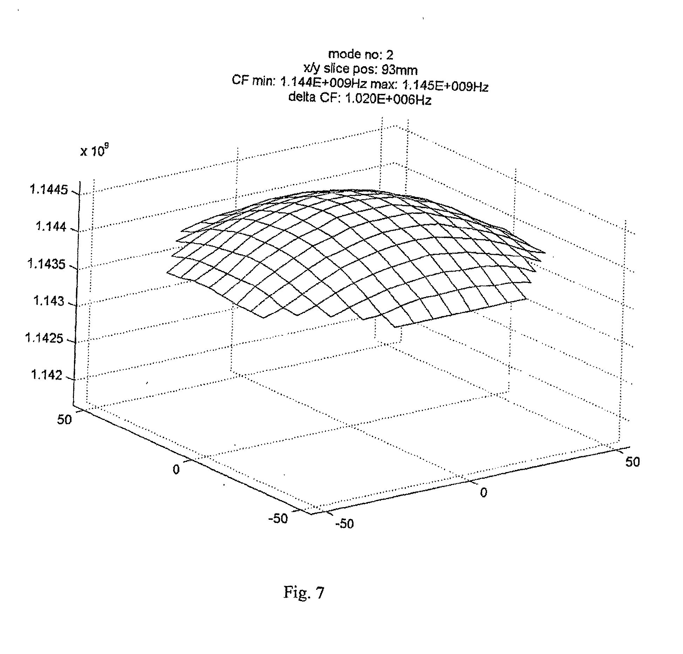

Fig. 7: Horizontaler Verlauf (entlang der X/Y-Ebene) der Resonanzfrequenz in der 2. Mode in der Höhe der Z-Achse von 93 mm. Horizontale Ebene der Figur entspricht den untersuchten Positionen in der X/Y-Achse.

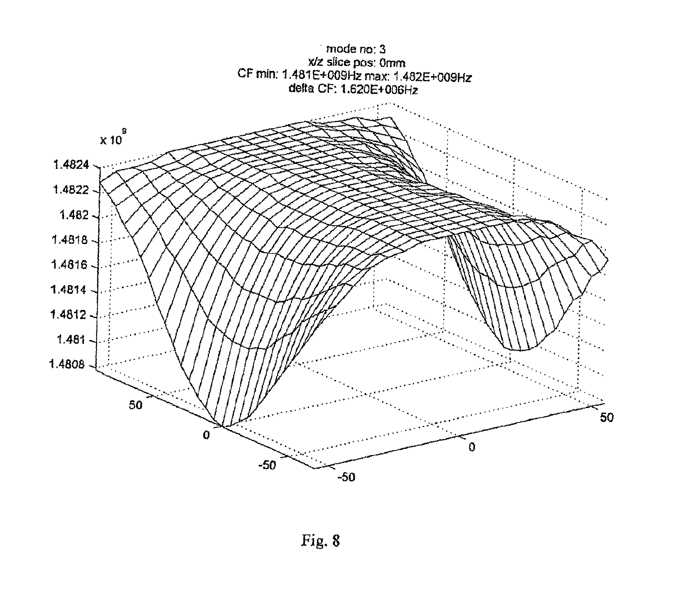

Fig. 8: Vertikaler Verlauf (entlang der Z-Achse) der Resonanzfrequenz in der 3. Mode. Horizontale Ebene der Figur entspricht den untersuchten Positionen in der X/Z-Achse.

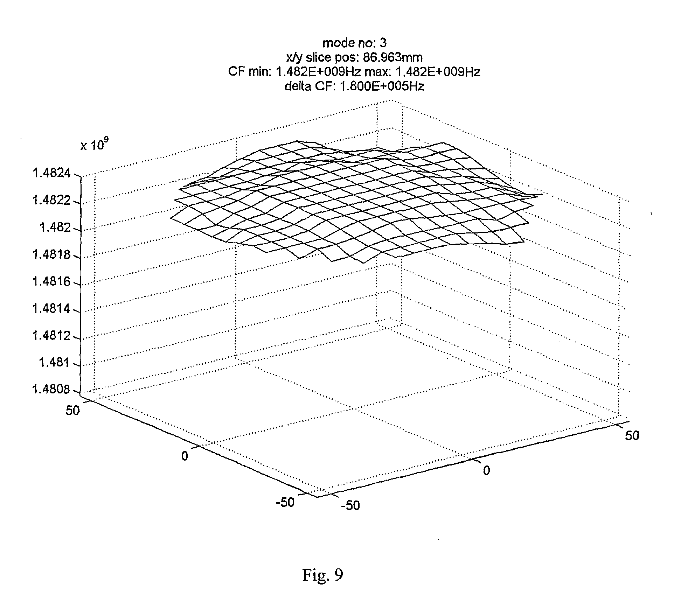

Fig. 9: Horizontaler Verlauf (entlang der X/Y-Ebene) der Resonanzfrequenz in der 3. Mode in der Höhe der Z-Achse von 87 mm. Horizontale Ebene der Figur entspricht den untersuchten Positionen in der X/Y-Achse.

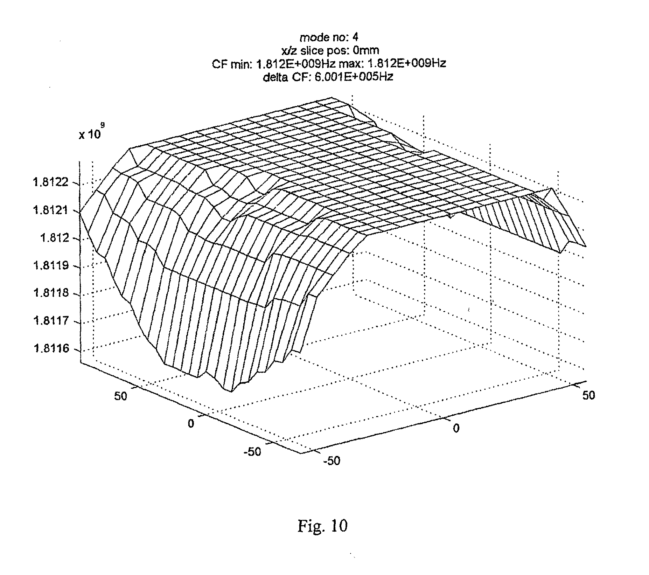

Fig. 10: Vertikaler Verlauf (entlang der Z-Achse) der Resonanzfrequenz in der 4. Mode. Horizontale Ebene der Figur entspricht den untersuchten Positionen in der X/Y-Achse.

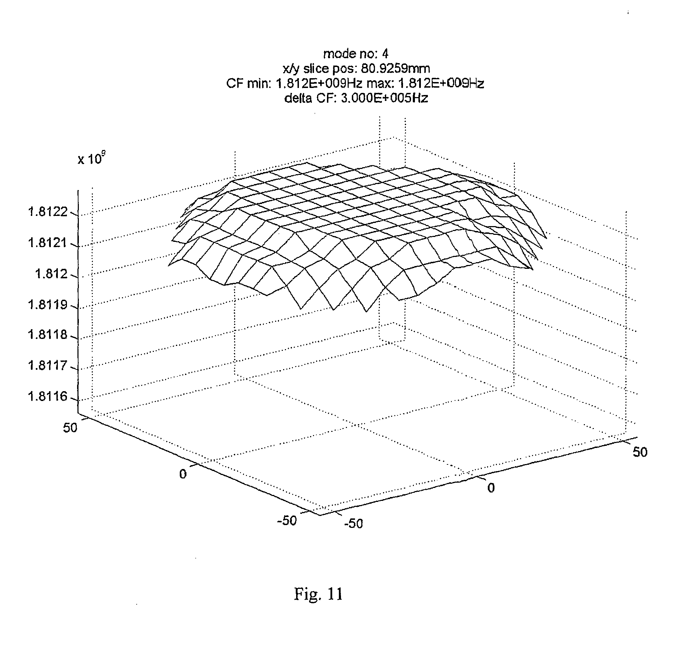

Fig. 11: Horizontaler Verlauf (entlang der X/Y-Ebene) der Resonanzfrequenz in der 4. Mode in der Höhe der Z-Achse von 80 mm. Horizontale Ebene der Figur entspricht den untersuchten Positionen in der X/Y-Achse.

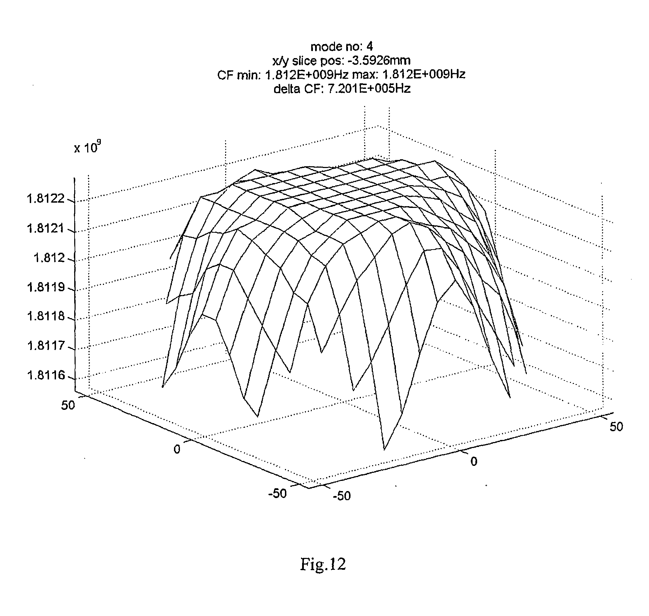

Fig. 12: Horizontaler Verlauf (entlang der X/Y-Ebene) der Resonanzfrequenz in der 4. Mode in der Höhe der Z-Achse von -4 mm. Horizontale Ebene der Figur entspricht den untersuchten Positionen in der X/Y-Achse.

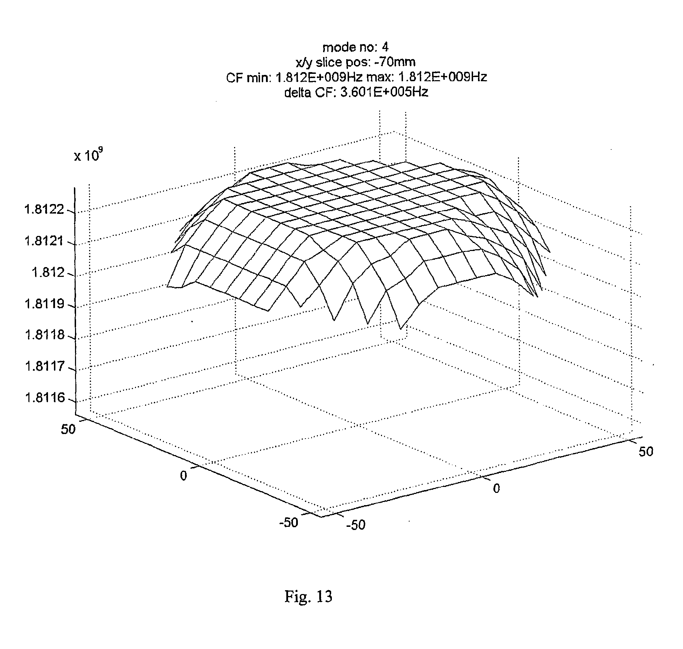

Fig. 13: Horizontaler Verlauf (entlang der X/Y-Ebene) der Resonanzfrequenz in der 4. Mode in der Höhe der Z-Achse von -70 mm. Horizontale Ebene der Figur entspricht den untersuchten Positionen in der X/Y-Achse.

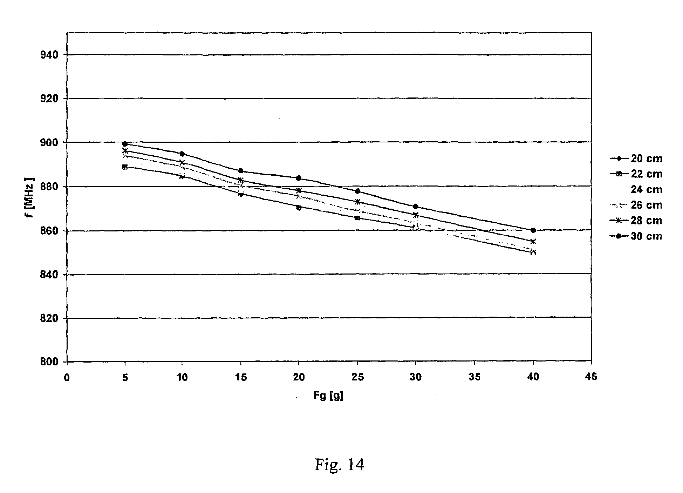

Fig. 14: Resonanzfrequenzen für verschiedene Feuchtgewichte [Fg] in Abhängigkeit von der Messposition. Ordinate X gibt das Feuchtgewicht [Fg] in (g) an. Abszisse Y gibt die Resonanzfrequenz [f] in (MHz) an.

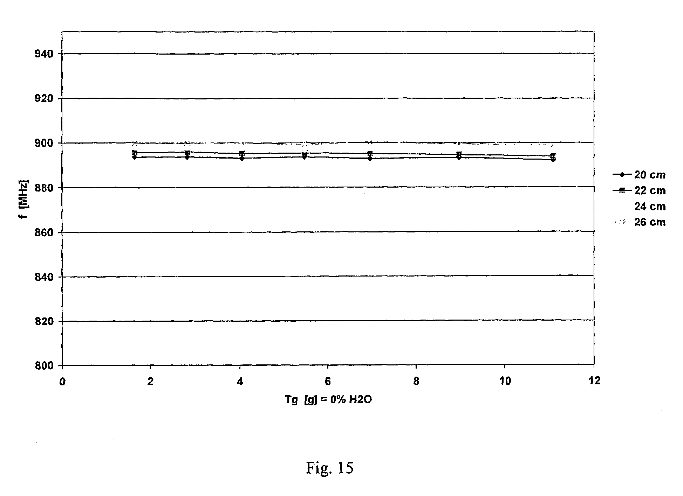

Fig. 15: Resonanzfrequenzen für verschiedene Trockengewichte [Tg] in Abhängigkeit von der Messposition im Resonator. Die Ordinate X gibt das Trockengewicht [Tg] in (g) an und die Abszisse Y die Resonanzfrequenz [f] in (MHz).

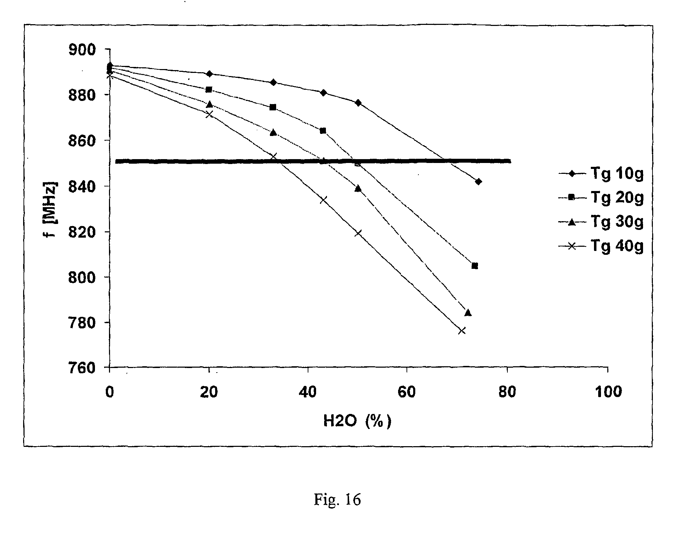

Fig. 16: Resonanzfrequenzen in Abhängigkeit vom prozentualen Wassergehalt nach kontrollierter Zugabe von Wasser zur getrockneten Biomasse mit definierter Ausgangstrockenmasse [TG] von 10g, 20g, 30g, 40g. Die Ordinate X gibt den prozentualen Wassergehalt $H_2O$ in (%) an und die Abszisse Y die Resonanzfrequenz [f] in (MHz).

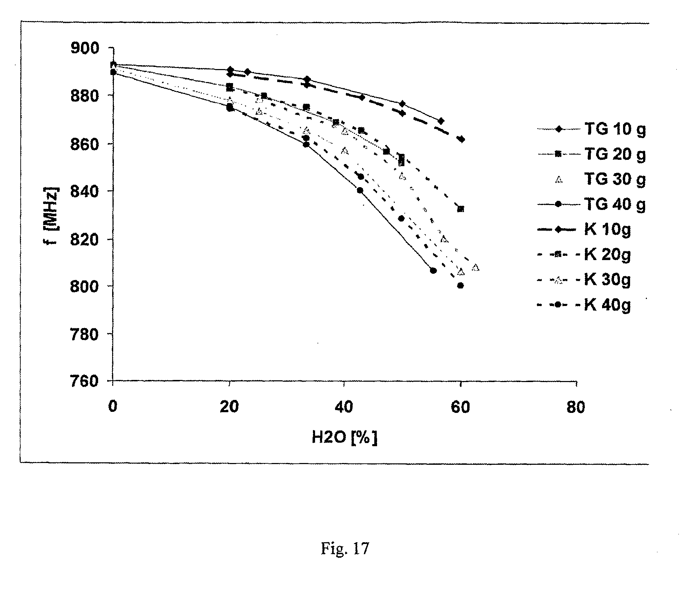

Fig. 17: Resonanzfrequenzen in Abhängigkeit vom prozentuellen Wassergehalt nach kontrollierter Zugabe von Wasser zur getrockneten Biomasse. Die Ordinate X gibt den Wassergehalt in % an und die Abszisse Y die Resonanzfrequenz [f] in (MHz). Als Kontrollmessung (K10g - K40g) wurden die Biomassewerte des erfindungsgemäßen Verfahrens mit den durch nach dem Stand der Technik bekannten Verfahren ermittelten Werten (TG10g- TG40g) verglichen.

Figur 1 zeigt beispielhaft die Geometrie eines Resonators mit (1) = Abschlussmanschetten, (2) = Resonatorraum, (3) = Einkopplungsschleifen, (4) Zuleitung zum Anschluss an den Analyseschwingkreis. Die möglichen Dimensionen sind in der folgenden Tabelle I aufgelistet. Die Ein- und Auskoppelschleifen werden für ein zirkular umlaufendes H- Feld orientiert, so dass die niedrigste Mode, die transversal- elektrische $TM_{010}$- Mode, bevorzugt angeregt wird. Die Resonanzfrequenz der Mode berechnet sich nach der Theorie von Hohlraumresonatoren in der Approximation eines vollständig geschlossenen Zylinders des Durchmessers $D_1$ und der Länge $L_1$ gemäß

$$f_{010}^E(D_1) = \frac{c}{\sqrt{\varepsilon_r \mu_r}} \cdot \frac{x_{01}}{\pi D_1} \ .$$

Dabei bezeichnet $x_{01}$ = 2.4048 die erste Nullstelle der Besselfunktion $J_0(x)$. Die VakuumLichtgeschwindigkeit ist c = 2.9979x $10^8$ m/s. $\varepsilon_r$ und $\mu_r$ beschreiben die dielektrische Suszeptibilität und die magnetische Permeabilität des Mediums im Resonator.

Tabelle I.: mögliche Dimensionen von Resonatoren

| Version | $D_1$ (mm) | $D_2$ (mm) | $L_1$ (mm) | $L_2$ (mm) | $f_{res}$(theo) (MHz) | $f_{res}$(exp) (MHz) |
|---|---|---|---|---|---|---|
| 1 | 190 | 190 | 275 | 125 | - | 898,4 |
| 2 | 300 | 180 | 210 | 126 | 765,0 | 812,8 |
| 3 | 250 | 150 | 175 | 100 | 917.9 | 976,9 |
| 4 | 180 | 130 | 90 | 65 | 1274.9 | 1428,6 |
| $f_{res}$ (theo) = Resonanzfrequenz theoretisch $f_{res}$ (exp) = Resonanzfrequenz experimentell Version 1 wurde für Biomassebestimmungen zur Silage eingesetzt (s.u. II.1). | | | | | | |

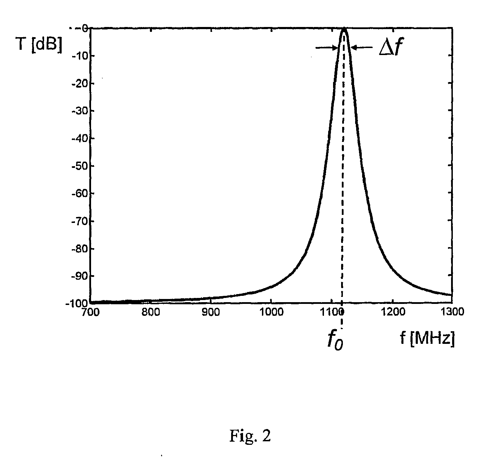

Figur 2 zeigt exemplarisch den Resonanzfrequenzverlauf der ersten Mode im Resonator mit den charakteristischen Resonanzeigenschaften wie Dämpfung, Güte und Lage. Die Abszisse X zeigt die Resonanzfrequenz [f] in (MHz). Die Ordinate Y zeigt die Transmission [T] in (dB). Die Güte/Qualität ergibt sich aus dem Quotient der Resonanzfrequenz [$f_0$] durch die Bandbreite $\Delta f$ bei $T_{min}$ -3dB.

Ausführungsbeispiele:

I. Kalibrierungsmessungen zur Bestimmung der Feldgeometrie eines Resonators

Der Versuchsaufbau bestand aus der Kombination XYZ-Verschiebetisch, Mikrowellenhohlraumresonator Version 1 aus Tabelle I (s. auch Fig 1) und Netzwerkanalysator (Rhode & Schwartz).

Zur Steuerung des Verschiebetisches und des Netzwerkanalysators wurden Anwendungsprogramme in LabVIEW (National Instruments) realisiert.

Der Netzwerkanalysator wurde derart programmiert, um in angegebenem Frequenzbereich die vollständige Transmissionskurve, Real- und Imaginärteil aufzuzeigen und zu sichern.

Die gemessenen und gespeicherten Daten wurden mit selbstprogrammierten MatLab Routinen ausgewertet (berechnet und graphisch dargestellt).

I. 1. Material und Methoden

Als Probekörper wurde ein Plexiglashohlwürfel (Kantenlänge: 2,5 cm, außen; 2,0 cm innen) gefüllt mit 7,3 g Wasser verwendet. Die Befestigung des Würfels am Verschiebetischarm erfolgte mittels eines Plexiglasstabes. Alle Messungen wurden bei Raumtemperatur (ca. 22 °C) durchgeführt.

Um die Messergebnisse an unterschiedlichen Positionen der Resonatorgeometrie und die Empfindlichkeit des Messverfahrens auf die Probenplatzierung zu testen, wurde der Probekörper im Resonatorinnenraum mit Hilfe eines 3-Achs-Verschiebetisches in XYZ-Richtung abgerastert.

I. 2. Die Verteilung der Moden

Die Verteilung der Moden ist abhängig von der Resonatorgeometrie, den Eigenschaften der elektromagnetischen Felder und der Dielektrizitätskonstante des untersuchten Mediums. Ein zylindrischer Hohlraumresonator mit einer bestimmten Form und Größe weist in einem vorgeschriebenen Frequenzband nur eine einzige Eigenschwingung, eine Mode, auf. Eine ausführliche Erklärung kann der Fachliteratur entnommen werden. ["Mikrowellen" A.J. Baden Fuller; "Mikrowellen" Gunter Nimtz].

Für die gegebene Resonatorgeometrie wurden die Resonanzfrequenzbereiche der Moden für die durchgeführten Messungen wie folgt festgelegt:

1. Mode von 750 bis ca. 900 MHz

2. Mode von 1150 bis 1350 MHz

3. Mode von 1450 bis 1650 MHz

4. Mode von 1750 bis 1950 MHz

Die Breite des Bereichs wurde dabei derart gewählt, dass typische Messungen mit und ohne Plexiglaszylinder nicht zu einer Verschiebung des Messsignals außerhalb des Bereichs führen, weil bei den Messungen mit Plexiglaszylinder sich die Verteilung der Moden zu niedrigeren Frequenzen verschiebt.

I. 3. Analyse der Daten auf die räumliche Homogenität

Für die Analyse auf die räumliche Homogenität wurden die Daten aus einer hoch aufgelösten Volumenmessung (3D) im Frequenzbereich von 700 MHz bis 1,9 GHz (mit Plexiglas Zylinder) zu Grunde gelegt. Die Messparameter waren dazu wie folgt:

| Objekt | Wassergefüllter Plexiglaswürfel |
|---|---|
| Maße | 25 mm$^3$ außen; 20 mm$^3$ innen |
| Wassermenge | 7,3 g |
| Startfrequenz | 700 MHz |
| Stopfrequenz | 1.9 GHz |
| Punktezahl im Spektrum | 20001 |
| Start- / Endkoordinaten | X = -55 bis +55 mm |
| | Y = -55 bis +55 mm |
| | Z = -70 bis 93 mm |
| Schrittweite | dx=dy=6.11 mm; z=6,037 mm |
| Wartezeit | 5 sec. |

I. 4. Ergebnisse

1. Mode:

Gemessene Resonanzfrequenz min. 775 MHz
Gemessene Resonanzfrequenz max. 776,5 MHz
Mittelwert: 775,75 MHz

Die Variierung der Resonanzfrequenz von unten nach oben entlang der Z-Achse in gesamten Messraum des Resonators beträgt 1,5 MHz. Dies entspricht ≤0.2 % zum Mittelwert. Der homogenste Bereich mit der Abweichung der Resonanzfrequenz von 0,1 % zum Mittelwert liegt vertikal von -70 bis +50 mm (entlang der Z-Achse) (s. Fig. 3) und horizontal von -45 bis +45 mm (045 mm) (entlang den X/Y Achsen)(s. Fig. 4 und 5).

Die max. Resonanzfrequenz Abweichung bei X/Y Flächen entlang der Z-Achse in gesamten Messraum beträgt 0,9 MHz, entspricht 0.12 % zum Mittelwert.

2. Mode:

Frequenzbereich von 1100 bis 1200 MHz
Gemessene Resonanzfrequenz min. 1142 MHz
Gemessene Resonanzfrequenz max. 1145,6 MHz
Mittelwert: 1143,8 MHz

Der homogene Frequenzbereich (Abweichung der Resonanzfrequenz ≤3 MHz, zum Mittelwert entspricht ≤0,26 %) liegt im gesamten Messraum im Resonator vertikal von -70 bis +93 mm (entlang der Z-Achse) (s. Fig. 6) und horizontal von -50 bis +50 mm (050 mm) (entlang den X/Y Achsen)(s. Fig.7).

Das minimale Rauschen (Abweichung der Resonanzfrequenz ≤1 MHz, entspricht ≤0,1%) befindet sich im oberen Messbereich ab 70 mm entlang der Z-Achse.

3. Mode:

Frequenzbereich von 1460 bis 1560 MHz
Gemessene Resonanzfrequenz min. 1480,8 MHz
Gemessene Resonanzfrequenz max. 1482,4 MHz
Mittelwert: 1481,6 MHz

Die Abweichung der Resonanzfrequenz der 3. Mode im gesamten Messraum des Resonators (min. Resonanzfrequenz in unterem Teil bei 1480,8 MHz; max. in oberem Teil bei 1482,4 MHz) beträgt 1,6 MHz und liegt somit unter 0,1 % zum Mittelwert. Die Stabilität entlang der Z-Achse (s. Fig. 8), wie auch bei weiteren (höheren) Moden ermöglicht die Messungen an höher wachsenden Pflanzen. Im engeren Bereich 0 20 mm erhält man sehr homogene Messungen. Die Resonanzfrequenz Abweichung liegt unter 0,02% zum maximalen Wert von 1482,4 MHz. Wie auch bei der 2. Mode nimmt das Rauschen im oberen Teil des Resonators ab (s. Fig. 9).

4.Mode:

Gemessene Resonanzfrequenz min. 1811,6 MHz
Gemessene Resonanzfrequenz max. 1812,2 MHz
Mittelwert: 1811,9 MHz

Die maximale Abweichung der Resonanzfrequenz der 4. Mode beträgt 0,6 MHz; ca. 0,03% zum Mittelwert. Somit erwies sich die 4. Mode als sehr stabil im gesamten Messbereich (s. Fig. 10 bis 13).

5. Mode:

Gemessene Resonanzfrequenz min. 2141,8 MHz
Gemessene Resonanzfrequenz max. 2142,2 MHz
Mittelwert: 2141 MHZ

Die maximale Variation der gemessenen Resonanzfrequenz der 5. Mode im gesamten Messraum im Resonator beträgt 0,4 MHz, entspricht 0,02%.

Mit den hier beschriebenen Messungen konnte gezeigt werden, dass mit den hier ausgewählten Bedingungen und Verfahrens-/Vorrichtungsmerkmalen automatisierte Messungen und Auswertungen durchgeführt werden können. Die durchschnittliche Messdauer von ca. 60 Sekunden pro Datenpunkt (je nach digitaler Auflösung der Messung) erlaubt in Zukunft eine schnelle Bestimmung der Biomasse. Die räumliche Stabilität der Resonanzfrequenzen (Abweichung von max. 1 %) ist insgesamt sehr gut.

II. Bestimmung der Biomasse von Maissilage

Für die ersten Kalibrierungsmessungen wurden zunächst nur folgende Messgrößen auf Magnitudenbasis erfasst bzw. errechnet:

- *Resonanzfrequenz $f_0$*: Resonanzfrequenz der ersten Mode [MHz]
- *Bandbreite $\Delta f$*: Peak - Breite bei 3dB Transmissionsverlust
- *Güte/Qualität Q = $f_0$ / $\Delta f$*

Figur 2 zeigt exemplarisch den Frequenzverlauf der Magnitude der ersten Mode im Resonator. Die Zugabe von Wasser in den Resonatorraum bewirkt sowohl eine Verschiebung der Resonanzlage, f, als auch eine Änderung der Güte. Die Verschiebung der Resonanz und/oder Änderung der Güte und damit der Wert der Resonanzfrequenz ist damit ein Maß für die Bestimmung des Wassergehaltes.

II. 1. Messaufbau & Kalibrierungsmessungen

Der Versuchsaufbau für den ersten Prototypen bestand aus einem Mikrowellenhohlraumresonator und einem Netzwerkanalysator (Rhode & Schwarz). Die Resonatordimensionierung (Maße: Höhe: 525 mm, Durchmesser 190 mm) wurde mit Hinblick auf die zu untersuchenden Pflanzen gewählt und mit Manschetten verschlossen, um ausreichende Modenstabilität auch bei größerer Befüllung zu gewährleisten. Ein Plexiglaszylinder mit Boden wurde zusätzlich eingesetzt, um Pflanzenteile besser handhaben zu können.

Zunächst wurde in Vorversuchen (s.oben) mit einem Probekörper (Plexiglashohlkörper, äußere Dimensionen 2,5 x 2,5 x 2,5 cm$^3$ -gefüllt mit 7,3 g $H_2O$) der räumliche Verlauf von Resonanzfrequenz und Güte im Resonatorraum bestimmt, um dort eine optimale Messposition festlegen zu können. Aus diesen ersten Kalibrierungsdaten konnte die Positionsabhängigkeit des Messsignals in erster Abschätzung bestimmt werden.

II. 2. Material und Methoden

Die frische Mais-Silage wurde zunächst mit unbekanntem Wassergehalt bei Frischgewichten von 5, 10, 15, 20, 25, 30 und 40g untersucht. Nach Trocknung bei 100°C für 24h wurden die Trockenmassen durch Wiegen auf 0,1g Genauigkeit bestimmt. Parallel wurden auch diese Proben an unterschiedlichen Positionen der Resonatorgeometrie untersucht, um die Empfindlichkeit des Messverfahrens auf die Probenplatzierung zu testen. Anschließend wurden definierte Trockenmassen mit Wasser auf definierte Feuchten (relative Wassergehalte (RWC)) angefeuchtet und untersucht, um eine Korrelation von RWC und Verschiebung der Resonanzfrequenz zu erstellen.

II. 3. Messergebnisse

Um eine möglichst genaue Aussage über die Empfindlichkeitsverteilung im Zylinder zu erhalten, wurde ein Bereich von 100 mm (auf 200 mm bis 300 mm der Bauhöhe) in 20 mm Abständen abgerastert. Figur 14 verdeutlicht, dass eine -wenn auch geringe- Abhängigkeit zwischen Messposition und Resonanzfrequenz besteht.

Bei Messung der getrockneten Mais-Silage konnte gezeigt werden, dass der Wert der Frequenzverschiebung bei entsprechender Füllmenge im Bereich von 200 bis 260 mm unabhängig von der Position des Messvolumens im Zylinder ist (Figur 15). Daraus lässt sich schließen, dass die Resonanzfrequenz im gewählten Probevolumen im Wesentlichen nur vom Feuchtegehalt in der Probe abhängt.

Wurde definierten Trockenmassen kontrolliert Wasser zugegeben, zeigen die resultierenden Resonanzfrequenzen Abhängigkeiten von Ausgangstrockenmasse und relativem Wassergehalt. Dieser ergibt sich aus

$$\text{Rel. Wassergehalt } [\%] = \text{Wasser } [g]/(\text{Trockenmasse } [g] + \text{Wasser } [g])$$

Dies war zu erwarten, da eine Änderung der Resonanzfrequenz direkt mit der absoluten Menge an zugegebenem Wasser korreliert. Eine horizontale Linie in Fig. 16 bei 850 MHz verdeutlich dies: Ein Ausgangstrockengewicht von 40 g mit einem relativen Wassergehalt von 33% zeigt die gleiche Resonanzfrequenz wie ein Ausgangstrockengewicht von 30 g mit einem relativen Wassergehalt von 40% oder auch wie ein Ausgangstrockengewicht von 20 g mit einem relativen Wassergehalt von 50%, nämlich eine Verschiebung der Resonanzfrequenz von 890 MHz wasserfrei auf ~ 850 MHz. In allen drei Fällen wurde die gleiche absolute Wassermenge von 20 g zugegeben.

Somit konnte gezeigt werden, dass mit Hilfe einer entsprechenden Kalibrierung, über die Verschiebung der Resonanzfrequenz in einer eingewogenen Silageprobe die absolute Menge an Wasser bestimmt werden kann. Ist diese dann bekannt, können Trockenmasse, relativer Wassergehalt und relativer Anteil Trockenmasse wie folgt berechnet werden:

| | |
|---|---|
| Trockenmasse (g) | = Einwaage (g)-Wasser(g) |
| Relativer Wassergehalt (%) | = Wasser (g) / Einwaage (g) * 100 |
| Relativer Anteil Trockenmasse (%) | = [Trockenmasse (g)/ Einwaage (g)] * 100 |

Um die Messgenauigkeit, bzw. den Fehler des Verfahrens abzuschätzen, wurden weitere (Blind-) Messungen mit Maissilage aus einer weiteren Lieferung durchgeführt. Es handelte sich um jeweils 4 Proben mit Trockengewichten von 10; 20; 30 und 40g von getrockneter Silage (0% Feuchtigkeit) zu denen nach und nach kontrolliert Wasser zugegeben wurde. Gemessen wurde im Frequenzbereich der 1. Mode von 713 MHz bis 1013 MHz, die Anzahl der Messpunkte im Spektrum betrug 201, siehe Fig. 17.

Bei allen Messungen ergaben sich sehr gute Korrelationen zwischen dielektrisch (Mikrowellen) und gravimetrisch bestimmtem Wassergehalt, lediglich mit kleinen (<0.5%, bei einem Datenpaar <1.8%) Abweichungen. Der Mittelwert der Abweichungen von drei Messserien zu verschiedenen Zeitpunkten und mit Silage Proben aus verschiedenen Lieferungen beträgt ca.0,5 %.

## Patentansprüche

1. Verfahren zur nicht- invasiven Bestimmung der Verteilung von Biomasse im Höhenprofil mittels dielektrischer Messungen im Mikrowellenhohlraumresonator,
   **dadurch gekennzeichnet,**
   **dass**

   - polarisierbare Biomasse von einem Mikrowellenhohlraumresonator umhüllt wird,
   - der Mikrowellenhohlraumresonator entlang der Pflanzenachse geführt wird,
   - die veränderten Resonanzeigenschaften ermittelt werden,
   - die Biomasse anhand von kalibrierten Daten ermittelt wird.

2. Verfahren nach Anspruch 1,
   **dadurch gekennzeichnet,**
   **dass** für jede Pflanzenart eine Kalibrierung durchgeführt wird und diese Daten für weitere Messungen verwendet werden.

3. Verfahren nach einem der Ansprüche 1 bis 2,
   **dadurch gekennzeichnet,**
   **dass** zur Bestimmung des Wassergehalts in einer niedrigen Mode des Resonators gemessen wird.

4. Verfahren nach einem der Ansprüche 1 bis 2,
   **dadurch gekennzeichnet,**

**dass** zur Bestimmung des Salzgehalts in einer höheren Mode des Resonators gemessen wird.

5. Verfahren nach einem der Ansprüche 1 bis 4,
   **dadurch gekennzeichnet,**
   **dass** das Pflanzsubstrat und/oder das Pflanzgefäß von der Biomasse, die untersucht werden soll, abgeschirmt wird.

6. Vorrichtung zur nicht-invasiven Bestimmung der Verteilung von Biomasse im Höhenprofil mittels dielektrischer Messungen im Mikrowellenhohlraumresonator, umfassend mindestens einen Mikrowellenhohlraumresonator mit Auswerteelektronik,
   **dadurch gekennzeichnet,**
   **dass** der Mikrowellenhohlraumresonator parallel zur Längsachse so aufklappbar ist, dass die Pflanze in den Mikrowellenhohlraumresonator eingeführt werden kann, der Mikrowellenhohlraumresonator entlang der Pflanzenachse verschiebbar ist und der gesamte Resonatorraum (2) als Mikrowellenhohlraumresonator ausgestaltet ist.

## Claims

1. Method for non-invasive determination of the distribution of biomass in the height profile by means of dielectric measurements in the microwave cavity resonator, **characterised in that**

   - polarisable biomass is covered by a microwave cavity resonator,
   - the microwave cavity resonator is guided along the plant stem,
   - the changed resonance characteristics are established,
   - the biomass is established using calibrated data.

2. Method according to claim 1,
   **characterised in that**
   calibration is carried out for every type of plant and this data is used for further measurements.

3. Method according to one of claims 1 to 2,
   **characterised in that**
   measurement is made in a low mode of the resonator to determine the water content

4. Method according to one of claims 1 to 2,
   **characterised in that**
   measurement is made in a high mode of the resonator to determine the salt content

5. Method according to one of claims 1 to 4,
   **characterised in that**
   the plant substrate and/or plant container is shielded from the biomass, which is to be examined.

6. Device for non-invasive determination of the distribution of biomass in the height profile by means of dielectric measurements in the microwave cavity resonator, comprising at least one microwave cavity resonator with evaluation electronics,
   **characterised in that**
   the microwave cavity resonator may be hinged parallel to the longitudinal axis so that the plant may be introduced into the microwave cavity resonator, the microwave cavity resonator may be moved along the plant stem and the whole resonator area (2) is made as a microwave cavity resonator.

## Revendications

1. Procédé de détermination non invasive de la répartition de biomasse dans le profil en hauteur au moyen de mesures diélectriques dans le résonateur à hyperfréquences,
   **caractérisé en ce**
   **que**

   - la biomasse polarisable est enveloppée par un résonateur à hyperfréquences,

- le résonateur à hyperfréquences est guidé le long de l'axe de la plante,
- les propriétés de résonance modifiées sont déterminées,
- la biomasse est déterminée à l'aide de données calibrées.

2. Procédé selon la revendication 1,
   **caractérisé en ce**
   **qu'**un calibrage est effectué pour chaque type de plante et que ces données sont employées pour d'autres mesures.

3. Procédé selon l'une quelconque des revendications 1 à 2,
   **caractérisé en ce**
   **que**, pour la détermination de la teneur en eau, la mesure est effectuée dans un mode bas du résonateur.

4. Procédé selon l'une quelconque des revendications 1 à 2,
   **caractérisé en ce**
   **que**, pour la détermination de la teneur en sel, la mesure est effectuée dans un mode plus haut du résonateur.

5. Procédé selon l'une quelconque des revendications 1 à 4,
   **caractérisé en ce**
   **que** le substrat de la plante et/ou le récipient de la plante est mis sous écran par la biomasse qui doit être examinée.

6. Dispositif de détermination non invasive de la répartition de biomasse dans le profil en hauteur au moyen de mesures diélectriques dans le résonateur à hyperfréquences, comportant au moins un résonateur à hyperfréquences avec une électronique d'évaluation, **caractérisé en ce**
   **que** le résonateur à hyperfréquences est relevable le long de l'axe longitudinal de sorte que la plante puisse être introduite dans le résonateur à hyperfréquences,
   **que** le résonateur à hyperfréquences est déplaçable le long de l'axe de la plante et que toute la cavité de résonance (2) forme le résonateur à hyperfréquences.

D2

(1) →

L2

(2) →

L1

(3) →

(4)

L2

D1

Fig. 1

Z

Y

X

Fig. 1a

Fig. 2

Fig. 3

mode no: 1
x/y slice pos: 50.7407mm
CF min: 7.754E+008Hz max: 7.761E+008Hz
delta CF: 6.600E+005Hz

Fig. 4

mode no: 1
x/y slice pos: -70mm
CF min: 7.749E+008Hz max: 7.757E+008Hz
delta CF: 7.800E+005Hz

Fig. 5

Fig. 6

mode no: 2
x/y slice pos: 93mm
CF min: 1.144E+009Hz max: 1.145E+009Hz
delta CF: 1.020E+006Hz

Fig. 7

Fig. 8

mode no: 3
x/y slice pos: 86.963mm
CF min: 1.482E+009Hz max: 1.482E+009Hz
delta CF: 1.800E+005Hz

Fig. 9

mode no: 4
x/z slice pos: 0mm
CF min: 1.812E+009Hz max: 1.812E+009Hz
delta CF: 6.001E+005Hz

Fig. 10

mode no: 4
x/y slice pos: 80.9259mm
CF min: 1.812E+009Hz max: 1.812E+009Hz
delta CF: 3.000E+005Hz

Fig. 11

mode no: 4
x/y slice pos: -3.5926mm
CF min: 1.812E+009Hz max: 1.812E+009Hz
delta CF: 7.201E+005Hz

Fig.12

mode no: 4
x/y slice pos: -70mm
CF min: 1.812E+009Hz max: 1.812E+009Hz
delta CF: 3.601E+005Hz

Fig. 13

Fig. 14

Fig. 15

Fig. 16

Fig. 17

**IN DER BESCHREIBUNG AUFGEFÜHRTE DOKUMENTE**

**In der Beschreibung aufgeführte Nicht-Patentliteratur**

- **KRASZEWSKI W. et al.** Microwave resonator for sensing moisture content and mass of single wheat kernels. *der Zeitschrift Canadian Agricultural Engineering,* 01. Oktober 1994, vol. 36 (4), 231-238